# EUROPÄISCHE PATENTSCHRIFT

(11) **EP 4 138 625 B1**
(45) Veröffentlichungstag und Bekanntmachung des Hinweises auf die Patenterteilung: **21.02.2024**
(21) Anmeldenummer: 21718872.1
(22) Anmeldetag: 14.04.2021
(51) Int. Cl.: A61B 1/00, A61B 1/267

(54) **MEDIZINISCHE VORRICHTUNG**
MEDICAL DEVICE
DISPOSITIF MÉDICAL

(30) Priorität: 21.04.2020 DE 102020110840
(43) Veröffentlichungstag der Anmeldung: 01.03.2023
(73) Patentinhaber: Freitag, Lutz, 58675 Hemer (DE)
(72) Erfinder: Freitag, Lutz, 58675 Hemer (DE)
(74) Vertreter: Griepenstroh, Jörg
(86) Internationale Anmeldenummer: PCT/EP2021/059673
(87) Internationale Veröffentlichungsnummer: WO 2021/213861

(56) Entgegenhaltungen:
- EP-A2- 2 097 721
- EP-B1- 2 097 721
- DE-U1-202020 102 272
- US-A1- 2009 143 996
- US-A1- 2017 258 550
- US-A1- 2018 279 860

## Beschreibung

Die Erfindung betrifft eine medizinische Vorrichtung mit den Merkmalen des Patentanspruchs 1.

Endoskope werden für minimalinvasive, operative Eingriffe an Menschen und Tieren sowie in der Technik zur Sichtprüfung schwer zugänglicher Hohlräume eingesetzt. Je nach Verwendungszweck kommen Endoskoprohre mit unterschiedlichen Durchmessern zum Einsatz. In der Bronchoskopie kommen oftmals starre Bronchoskoprohre aus Edelstahl mit Wanddicken von 0,6 mm zum Einsatz. Durchmesser im Bereich von 10 mm mit Längen von ca. 400 mm sind üblich. Zum Platzieren der häufig verwendeten Silikon Stents sind Rohrdurchmesser von 10-16 mm erforderlich entsprechend der Weite eines normalen Kehlkopfes. Der Umgang mit dickeren Geräten zur Stenteinlage erfordert Übung und Erfahrung.

Ein weiteres Problem besteht darin, dass bei der Endoskopie in der Regel nicht ohne größeren Aufwand von einem kleineren Durchmesser auf einen größeren Durchmesser oder umgekehrt gewechselt werden kann. In der Regel ist es hierzu erforderlich, das Endoskoprohr einschließlich des daran befestigten Endoskopkopfes komplett zu tauschen und alle darin angeordneten Anschlüsse neu zu befestigen. Zu den Anschlüssen zählen bei der Bronchoskopie insbesondere Anschlüsse zur Ventilation der Lunge und auch Anschlüsse zur Messung der Druckverhältnisse innerhalb des Endoskops.

Die EP 2 097 721 A2 offenbart eine medizinische Vorrichtung mit einem Endoskopkopf, der dazu ausgebildet ist, mit einem Endoskoprohr lösbar verbunden zu werden. In dem Endoskopkopf ist ein Messkanal angeordnet, der eine innere Anschlussöffnung aufweist, die an einen Längskanal des Endoskoprohrs angeschlossen ist. Eine äußere Anschlussöffnung ist außenseitig des Endoskopkopfes angeordnet, wobei die äußere Anschlussöffnung mit wenigstens einem Messkanal in den Messadapter verbindbar ist. Der Messadapter ist lösbar mit dem Endoskopkopf koppelbar und als U-förmige Klammer konfiguriert, die einen Rücken und zwei Arme besitzt. Der Messadapter wird aufgesteckt und über federnde Klemmzungen fixiert. Er ist im übrigen formstabil.

Die US 2018/279860 A1 offenbart eine weitere medizinische Vorrichtung mit einem Endoskopkopf, der dazu ausgebildet ist, mit Endoskoprohren verbunden zu werden. Auch dieser Endoskopkopf weist einen Messkanal auf mit einer inneren Anschlussöffnung, die an den Längskanal des Endoskoprohrs angeschlossen ist. Weitere Bauformen von Endoskopen sind durch die US 2017/258550 A1 und US 2009/143996 A1 bekannt geworden.

Der Erfindung liegt die Aufgabe zugrunde, eine medizinische Vorrichtung mit einem Endoskopkopf aufzuzeigen, der es ermöglicht, auf einfache Weise unterschiedliche Endoskoprohre zu verwenden und möglichst unkompliziert die notwendigen Anschlüsse mit dem Endoskopkopf herzustellen.

Diese Aufgabe ist bei einer medizinischen Vorrichtung mit den Merkmalen des Patentanspruchs 1 gelöst.

Die Unteransprüche betreffen vorteilhafte Weiterbildungen der Erfindung.

Die medizinische Vorrichtung umfasst einen Endoskopkopf, der dazu ausgebildet ist, mit Endoskoprohren lösbar verbunden zu werden. Hierzu besitzt der Endoskopkopf einen Längskanal zwischen seinem proximalen Ende und seinem distalen Ende. Zusätzlich ist in dem Endoskopkopf wenigstens ein Messkanal angeordnet. Der wenigstens eine Messkanal besitzt wenigstens eine innere Anschlussöffnung, die an den Längskanal angeschlossen ist, und wenigstens eine äußere Anschlussöffnung, die außenseitig des Endoskopkopfes angeordnet ist. Die äußere Anschlussöffnung kann insbesondere lateral angeordnet sein. Vorzugsweise ist der Messkanal im Endoskopkopf so kurz wie möglich. Er verläuft daher bevorzugt radial. Vorzugsweise sind zwei diametral angeordnete, radial verlaufende Messkanäle im Endoskopkopf vorgesehen.

Die äußere Anschlussöffnung des wenigstens einen Messkanals ist mit einem Messadapter verbindbar. Der Messadapter ist lösbar mit dem Endoskopkopf koppelbar. Der Messadapter dient als Bindeglied und insbesondere als Schnellkupplung zwischen einem Messgerät, das beispielsweise über einen Schlauch mit dem Messadapter verbunden ist und dem Endoskopkopf. Die Verbindung der einzelnen Messkanäle ist insbesondere gasleitend. Die Erfindung hat den Vorteil, dass auch bei mehreren Messkanälen im Endoskopkopf diese mit einer einzigen Steckbewegung mit dem Messadapter verbunden werden können. Es ist nicht erforderlich, jeden Messkanal einzeln anzuschließen. Dadurch ist es möglich, den Endoskopkopf auf einfache Weise und unkompliziert für seine Verwendung vorzubereiten.

Der Messadapter soll vorzugsweise besonders einfach und schnell mit dem Endoskopkopf gekoppelt werden können und genauso einfach wieder entfernt werden können. Er ist über eine Steckverbindung mit dem Endoskopkopf koppelbar. Die Verbindung kann ohne Werkzeuge hergestellt werden. Der Messadapter ist als U-förmige Klammer konfiguriert. Er besitzt zwei Arme, die über einen Rücken als Bindeglied miteinander verbunden sind. Eine solche U-förmige Klammer kann lateral auf den Endoskopkopf gesteckt werden. Das seitliche Aufstecken ist besonders einfach und schnell möglich. Durch geeignete Kontakt- und Führungsflächen ist eine Fehlbedienung oder ein Verrutschen des Messadapters relativ zum Endoskopkopf ausschließbar.

Der Messadapter wird unter Ausnutzung einer Klemmkraft an dem Endoskopkopf gehalten. Hierzu sind die beiden Arme federnd ausgebildet. Insbesondere besteht der gesamte Messadapter aus einem federnden Werkstoff, sodass aufgrund der Materialeigenschaften beide Arme federnd ausgebildet sein können. Die Federkraft kann zusätzlich durch eine elastische Verformung des Rückens aufgebaut werden. Die Arme werden beim lateralen Aufstecken auf den Endoskopkopf leicht auseinandergebogen. Vorzugsweise sind an dem Endoskopkopf und/oder an den freien Enden der Arme Schrägflächen angeordnet, die aufeinander abgleiten, so dass die Klammer aufgebogen wird. Ist die Klammer in der finalen Position, federn die Arme zurück und liegen klemmend und dichtend an dem Endoskopkopf.

Die Verbindung ist klemmend, d. h. kraftschlüssig und formschlüssig. Eine formschlüssige Verbindung in Aufsteckrichtung bzw. entgegen der Aufsteckrichtung verhindert bei entsprechend gewählten Toleranzen das Verrutschen des Messadapters und damit eine Fehlstellung, eine Fehlbedienung bzw. ein Verfälschen von Messwerten. Es ist daher vorgesehen, dass beide Arme Rastvorsprünge aufweisen, mittels welcher der Messadapter formschlüssig mit dem Endoskopkopf koppelbar ist. Ein Formschluss in Aufsteckrichtung kann bereits dadurch hergestellt werden, dass der Rücken einer U-förmigen Klammer an einem Anlageabschnitt des Endoskopkopfes zur Anlage gelangt. Dadurch kann die Aufstecktiefe begrenzt werden. Seitliche Führungen für einen oder beide Arme an den Armen und/oder an dem Endoskopkopf stellen die Lage des Messadapters in Längsrichtung des Endoskopkopfes sicher.

Die Rastvorsprünge befinden sich im Abstand vom Rücken. Dadurch kann in Kombination mit der Federkraft der Arme ein überwiegendes Umgreifen des Endoskopkopfes sichergestellt werden. Ein vollständiges Umgreifen ist möglich, jedoch nicht zwingend erforderlich. Die Rastvorsprünge befinden sich insbesondere an den freien Enden der Arme. Beim Aufstecken des Messadapters werden die Arme mit ihren nach innen gerichteten Rastvorsprüngen ein wenig auseinandergebogen. Die Rastvorsprünge gleiten über die Außenseite des Endoskopkopfes und schnappen schließlich hinter eine Rastkante, sodass die Arme zurückfedern, und sodass eine hinreichend dichte Verbindung zwischen dem Messkanal oder den Messkanälen im Endoskopkopf und dem Messkanal bzw. den Messkanälen im Messadapter hergestellt wird. Grundsätzlich ist es auch möglich, die Federkraft der Arme nur dafür auszunutzen, dass in Kombination mit den Rastvorsprüngen eine formschlüssige Verbindung in der Einrastposition hergestellt wird. Als besonders günstig wird es angesehen, wenn es sich um eine Kombination aus formschlüssige Verbindung zur Lageorientierung und kraftschlüssiger Verbindung zur Abdichtung des Überganges im Bereich der äußeren Anschlussöffnung handelt.

Der Messadapter ist wie vorstehend erläutert dazu ausgebildet, in einem lateralen Adapteranschlussbereich des Endoskopkopfes angeordnet zu werden, wobei der Messadapter quer zum Längskanal auf den Adapteranschlussbereich schiebbar ist. Die Rastvorsprünge an den freien Enden der Arme umgreifen dabei mindestens zwei Drittel des Endoskopkopfes, bezogen auf seinen Durchmesser, wodurch eine sichere Führung und Fixierung des Messadapters gegeben ist.

Im Messadapter befindet sich wenigstens ein Messkanal, der im Bereich des Rückens des Messadapters mündet, insbesondere im Übergangsbereich zu den Armen. Im Bereich des Rückens ist hierzu insbesondere ein Anschlussstutzen vorgesehen, auf den insbesondere ein Schlauch aufgesteckt werden kann. Es ist mindestens ein Messkanal in einem der Arme des Messadapters angeordnet. Vorzugsweise ist in jedem der Arme des Messadapters ein Messkanal angeordnet, der wiederum jeweils mit einer äußeren Anschlussöffnung des Messkanals im Endoskopkopf koppelbar ist. Daher befinden sich an dem Rücken bevorzugt zwei Anschlussstutzen, die von dem Endoskopkopf wegweisen. Mehrere Messkanäle je Arm sind ebenso möglich.

Der Endoskopkopf ist dazu ausgebildet, mit Endoskoprohren lösbar verbunden zu werden. Damit der Messkanal im Endoskopkopf auch an entsprechende Messkanäle in dem Endoskoprohr angeschlossen werden kann, muss das Endoskoprohr in einer bestimmten Position im Endoskopkopf lageorientiert werden. Zuverlässig ist dies über eine formschlüssige Verbindung möglich. Eine formschlüssige Verbindung zwischen Endoskoprohr und Endoskopkopf hat zudem den Vorteil, dass über den Endoskopkopf eine Kraft auf das Endoskoprohr ausgeübt werden kann, beispielsweise um dieses in die richtige Position zu drehen. Daher ist der Endoskopkopf in vorteilhafter Weise derart mit dem Endoskoprohr gekoppelt, dass die Verbindung verdrehsicher ist. Die Verdrehsicherung kann kraftschlüssig und/oder formschlüssig ausgebildet sein. Vorzugsweise ist die Verbindung formschlüssig oder primär formschlüssig. Der Formschluss kann dadurch hergestellt werden, dass ein Vaterstück mit einem passenden Mutterstück als Gegenstück kombiniert wird. Mindestens ein Vaterstück kann hierzu am Endoskoprohr und/oder Endoskopkopf mit wenigstens einem Mutterstück am Endoskoprohr und/oder am Endoskopkopf in Eingriff gebracht werden. Es handelt sich im einfachsten Fall um eine oder mehrere Nut-Feder-Verbindungen.

Eine solche formschlüssige Verbindung kann gleichzeitig die Einstecktiefe des Endoskoprohrs in den Endoskopkopf bestimmen, sodass laterale Abschlussöffnungen der Messkanäle am Endoskoprohr mit den entsprechenden Anschlussöffnungen im Endoskopkopf korrespondieren.

In einer Weiterbildung der Erfindung ist es vorgesehen, dass das endoskoprohrseitige Vaterstück oder Mutterstück an einer Adapterhülse ausgebildet ist, die mit einem proximalen Ende des Endoskoprohrs koppelbar ist. Die Adapterhülse besitzt die notwendigen Formschlusselemente für eine verdrehfeste Verbindung mit dem Endoskopkopf. Die Adapterhülse begrenzt die Einstecktiefe des Endoskoprohrs in den Endoskopkopf und legt die Lage der Messkanäle fest. Auch die Adapterhülse besitzt einen oder mehrere Messkanäle, die an die Messkanäle in den Endoskoprohren anschließbar sind und beim Eingriff in den Längskanal des Endoskopkopfes mit dem dortigen Messkanal verbindbar sind.

In einer Weiterbildung der Erfindung sind Adapterhülsen mit aufeinander abgestimmten Durchmessern vorgesehen, sodass eine im Durchmesser kleinere Adapterhülse in eine im Durchmesser größere Adapterhülse steckbar ist. Die Adapterhülsen besitzen kraft- und/oder formschlüssige Verbindungen miteinander und zu den Bauteilen, mit denen sie in Eingriff gelangen. Durch derartige Adapterhülsen wird sichergestellt, dass eine Kraft vom Endoskopkopf über die Adapterhülsen und insbesondere über mindestens zwei Adapterhülsen auf ein angeschlossenes Endoskoprohr übertragen werden kann. Insbesondere ist es möglich, ein Endoskoprohr kleineren Durchmessers, das mit der kleineren Adapterhülse gekoppelt ist, über die als Zwischenhülse dienende Adapterhülse ebenfalls mit dem Endoskopkopf zu verbinden. Auf diese Weise ist es möglich, mit ein- und demselben Endoskopkopf unterschiedlich dicke Endoskoprohre zu verwenden, wobei der Dickenausgleich über die Verwendung passender Adapterhülsen erfolgt.

Alternativ zu Adapterhülsen, die mit unterschiedlichen Durchmessern ineinander gesteckt werden, aber jeweils auf den Durchmesser des Endoskoprohrs angepasst sind, ist es im Rahmen der Erfindung auch möglich, unterschiedliche Adapterhülsen zu verwenden, die einen ersten Durchmesserbereich zur Kopplung mit dem Längskanal des Endoskoprohrs besitzen und einen zweiten, gegebenenfalls kleineren Durchmesserbereich, zur Verbindung mit dem Endoskoprohr abweichenden Durchmessers.

Entsprechend der Konfiguration des Endoskoprohrs bzw. des Endoskopkopfes sind die Messkanäle in den Adapterhülsen angeordnet, d.h. insbesondere diametral. Die Länge der Messkanäle ist möglichst kurz. Die Anordnung der Messkanäle ist daher vorzugsweise nicht nur diametral, sondern auch radial.

Bei dem verwendeten Endoskop handelt es sich insbesondere um ein Bronchoskop.

Die Erfindung wird nachfolgend anhand eines in schematischen Zeichnungen dargestellten Ausführungsbeispiels erläutert.

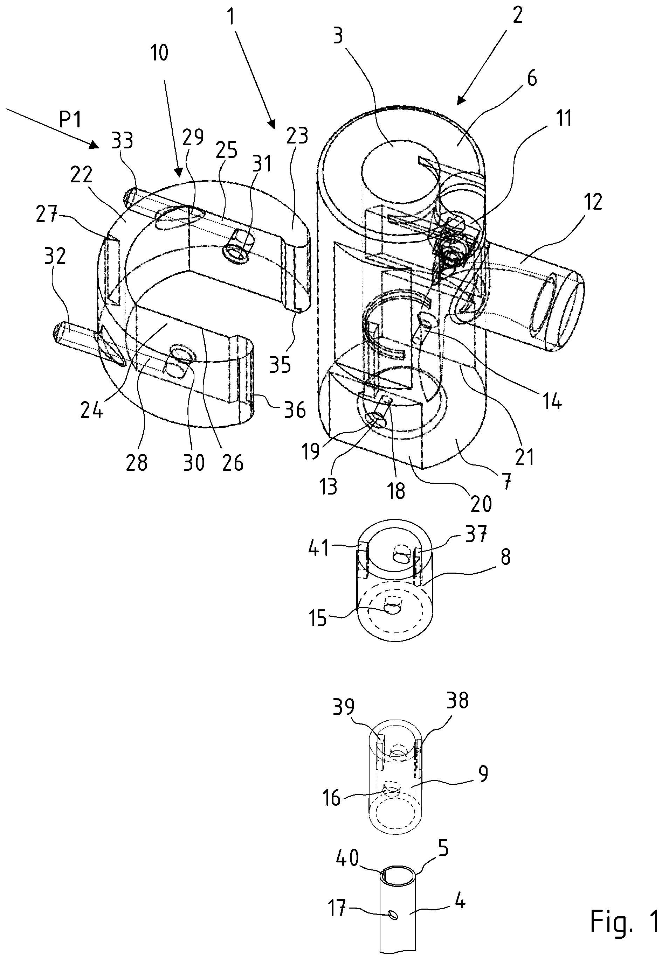

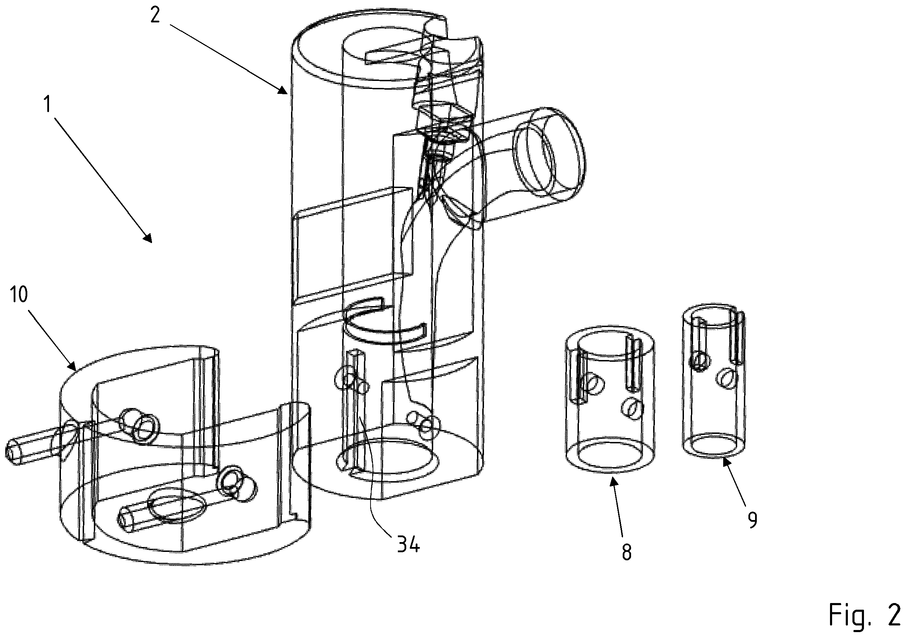

Die Figuren 1 und 2 zeigen zwei unterschiedliche perspektivische Darstellungen ein- und derselben medizinischen Vorrichtung 1 mit einem Endoskopkopf 2 als zentralen Bestandteil. Der Endoskopkopf 2 besitzt einen in der Bildebene vertikal verlaufenden Längskanal 3 zur Aufnahme eines Endoskoprohres 4. Das Endoskoprohr 4 ist nur teilweise dargestellt. Die Figur 1 zeigt nur dessen proximales Ende 5. Der Längskanal 3, der sich von einem proximalen Ende 6 bis zu einem distalen Ende 7 des Endoskopkopfes 2 erstreckt, dient zur Aufnahme des proximalen Endes 5 des Endoskoprohres 4. Der Durchmesser des Endoskoprohres 4 ist deutlich kleiner als der Innendurchmesser des Längskanals 3. Zum Ausgleich der Durchmesser werden Adapterhülsen 8, 9 verwendet, auf die weiter unten eingegangen wird.

Eine weitere Komponente der Vorrichtung 1 ist ein Messadapter 10. Der Messadapter 10 ist lösbar mit dem Adapterkopf 2 koppelbar, indem er seitlich in Richtung des Pfeils P1 auf den im Wesentlichen zylindrischen Endoskopkopf 2 gesteckt wird. Er kann in die Gegenrichtung wieder abgezogen werden. Es ist für das Aufstecken und Abnehmen kein Werkzeug erforderlich.

Der Endoskopkopf besitzt neben dem eigentlichen Längskanal 3, der als Arbeitskanal dient, Anschlüsse zur Ventilation. Diese Anschlüsse sind bei der Verwendung des Endoskopkopfes während einer Bronchoskopie vorgesehen. Ein erster Anschluss 11 dient zur Jet-Ventilation. Ein zweiter Anschluss 12 ist für eine übliche Ventilation mit niedrigeren Drücken vorgesehen. Die Anschlüsse 11, 12 können alternativ verwendet werden.

Zusätzlich besitzt der Endoskopkopf Messkanäle 13, 14. Die Messkanäle 13, 14 sind diametral angeordnet. Sie befinden sich in der Nähe des distalen Endes 7, sodass sie mit Messkanälen 15, 16 in den Adapterhülsen 8, 9 in Überdeckung gebracht werden können bzw. an einen Messkanal 17 im Endoskoprohr 4 angeschlossen werden können. Die Messekanäle 13, 14 münden in den Längskanal 3 und besitzen jeweils eine innere Anschlussöffnung 18 und eine äußere Anschlussöffnung 19. Die äußere Anschlussöffnung 19 befindet sich in einem lateralen Adapteranschlussbereich 20. Der Adapteranschlussbereich 20 ist abweichend von der übrigen Außenkontur des im Wesentlichen zylindrischen Endoskopkopfes 2 nicht gerundet, sondern abgeflacht. Da sich entsprechende Anschlussöffnungen 19 außenseitig diametral gegenüber liegen, sind auch die entsprechenden Adapteranschlussbereiche 20, 21 gegenüberliegend angeordnet. Die Adapteranschlussbereiche 20 sind abgeflacht und verlaufen in diesem Fall parallel zueinander. Über diese abgeflachten Adapteranschlussbereiche 20 kann der Messadapter 10 in Richtung des Pfeils P1 geschoben werden. Hierzu ist der Messadapter als U-förmige Klammer konfiguriert. Er besitzt einen Rücken 22 und zwei Arme 23, 24, die mit dem Rücken 22 verbunden sind. Die Arme 23, 24 sind federnd konfiguriert und dazu ausgebildet, mit ihren jeweils innen an den Armen ausgebildeten Kontaktflächen 25, 26 mit dem Adapteranschlussbereich 20, 21 in Kontakt zu kommen, zumindest aber eine fluidleitende, insbesondere gasleitende Verbindung zu den äußeren Anschlussöffnungen 19 der Messkanäle 13, 14 im Endoskopkopf 2 herzustellen. Die Arme 23, 24 haben einen gegenseitigen Abstand, der auf den Abstand der Adapteranschlussbereiche 20 angepasst ist.

Für die Federwirkung der Arme 23, 24 ist im Rücken 22 außenseitig eine Kerbe 27 angeordnet. Dadurch sind die Arme 23, 24 zwar in der Lage, nach außen aufzufedern, jedoch erfolgt die Federwirkung aufgrund einer Verformung im Bereich des Rückens 22. Die Arme 23, 24 selbst müssen nicht elastisch gestaltet sein, können es aber sein.

Die Messkanäle 13, 14 aus dem Endoskopkopf 2 werden durch weitere Messkanäle 28, 29 in dem Messadapter 10 fortgesetzt. In Längsrichtung der Arme 23, 24 verlaufend befindet sich dort jeweils ein Messkanal 28, 29. Die beiden Messkanäle 28, 29 besitzen jeweils Anschlussöffnungen 30, 31 in den einander zugewandten Kontaktflächen 25, 26. Die Messkanäle 28, 29 sind daher innerhalb der Arme 23, 24 abgewinkelt Diese Anschlussöffnungen 30, 31 korrespondieren in der aufgesteckten Position des Messadapters 10 mit den Anschlussöffnungen 19 am Endoskopkopf 2. Die Messkanäle 28, 29 in dem Messadapter 10 münden jeweils in Anschlussstutzen 32, 33, an die jeweils ein Schlauch angeschlossen werden kann, um den Druck am Messadapter 10 abzugreifen und auszuwerten.

Der Messadapter 10 kann durch nicht näher dargestellte Dichtmittel im Übergangsbereich zwischen den Messkanälen 28, 29 des Messadapters zu den Messkanälen 13, 14 im Endoskopkopf 2 abgedichtet sein. Das Abdichten kann auch über eine hinreichend groß gestaltete Spaltdichtung erfolgen, bspw. dadurch, dass die Kontaktflächen 25, 26 klemmend auf den Adapteranschlussbereichen 20, 21 aufliegen. Zur zusätzlichen Arretierung sind an den freien Enden der Arme jeweils nach innen gerichtete Rastvorsprünge 35, 36 vorgesehen. Hierbei handelt es sich um wulstartige Verdickungen, welche in der aufgesteckten Position hinter die abgeflachten lateralen Adapteranschlussbereiche 20 am Endoskopkopf 2 fassen und dadurch ein unbeabsichtigtes Abziehen des Messadapters 10 verhindern.

Der Rücken 22 ist gebogen und an die Außenkontur des Endoskopkopfes 2 angepasst. Er dient als Anschlag und begrenzt die Aufstecktiefe des Messadapters 10.

Es ist eine wesentliche Voraussetzung für eine Druckmessung, dass die Messkanäle in den jeweiligen Bauteilen frei durchgängig sind. Hierzu ist es erforderlich, die Messöffnung 17 im Endoskoprohr 4 so auszurichten, dass sie durchgängig mit dem Messkanal 13, 14 im Endoskopkopf 2 verbunden ist. Die exakte Positionierung wird durch die Verwendung der Adapterhülsen 8, 9 sichergestellt. Bei diesem Ausführungsbeispiel kommen exemplarisch verschieden große Adapterhülsen 8, 9 zum Einsatz. Die größere der beiden Adapterhülsen 8 besitzt einen Außendurchmesser, der an den Innendurchmesser des Längskanals 3 angepasst ist, d.h. dort hineingesteckt werden kann. Diese Adapterhülse 8 besitzt neben dem dort angeordneten Messkanal 15 zusätzlich einen nach innen gerichteten Radialvorsprung, der als Vaterstück 37 für ein Mutterstück 38 an der kleineren Adapterhülse 9 dient. Das Mutterstück 38 ist als radial außenseitige, nutförmige Vertiefung ausgebildet. Das Mutterstück 38 erstreckt sich nicht über die gesamte axiale Länge der Adapterhülse 9, so dass durch die Länge des Mutterstücks 38 die Einstecktiefe in die größere Adapterhülse 8 begrenzt und auch definiert werden kann. Auch die kleinere Adapterhülse 9 besitzt ein radial nach innen gerichtetes Vaterstück 39 in Form eines radial nach innen weisenden Längssteges. Dieses Vaterstück 39 soll in ein entsprechendes Mutterstück 40 in Form einer Längskerbe beginnend am proximalen Ende 5 des Endoskoprohrs 4 eingreifen. Auch hier wird durch die Länge des Vaterstücks 39 bzw. Mutterstücks 40 die exakte Position des Endoskoprohrs 14 in der kleineren Adapterhülse 9 bestimmt. Das Endoskoprohr 4 kann nun zusammen mit der kleineren Adapterhülse 9 und unter Eingliederung der größeren Adapterhülse 8 in den Endoskopkopf 2 eingeführt werden. Die größere Adapterhülse 8 besitzt zur Lageorientierung und zur Verdrehsicherung ein Mutterstück 41 in Form einer sich von ihrem proximalen Ende in Längsrichtung erstreckenden Längsnut. Diese Längsnut bzw. dieses Mutterstück 41 korrespondiert mit einem entsprechenden Steg in Form eines Vaterstücks 34 (Figur 2) im Inneren des Längskanals 3. Auf diese Weise ist eine Verdrehsicherung der Bauteile zueinander gewährleistet. In nicht näher dargestellter Weise kann die Anordnung aus Adapterhülsen 8, 9 und Endoskoprohr 4 zusätzlich noch gegen Verschiebung in Längsrichtung des Endoskoprohrs 4 gesichert werden. Hierbei kann es sich um eine Klemmverbindung handeln, die von radial außen auf das eingesetzte Endoskoprohr 4 einwirkt und dadurch die Verbindung zum Endoskopkopf 2 herstellt.

Die medizinische Vorrichtung 1 der Figur 1 ermöglicht es, auch größere Endoskoprohre 4 zu verwenden. Durch Weglassen der kleineren Adapterhülse 9 kann in gleicher Weise, wie es in der Figur 1 dargestellt ist, auch ein im Durchmesser größeres Endoskoprohr unmittelbar mit der größeren der Adapterhülsen 8 verbunden werden.

### Bezugszeichen:

- 1 -: medizinische Vorrichtung
- 2 -: Endoskopkopf
- 3 -: Längskanal
- 4 -: Endoskoprohr
- 5 -: proximales Ende von 4
- 6 -: proximales Ende von 2
- 7 -: distales Ende von 2
- 8 -: Adapterhülse
- 9 -: Adapterhülse
- 10 -: Messadapter
- 11 -: Anschluss für Jetventilation
- 12 -: Anschluss zur Ventilation
- 13 -: Messkanal in 2
- 14 -: Messkanal in 2
- 15 -: Messkanal in 8
- 16 -: Messkanal in 9
- 17 -: Messöffnung in 4
- 18 -: innere Anschlussöffnung von 13
- 19 -: äußere Anschlussöffnung von 13
- 20 -: Adapteranschlussbereich an 2
- 21 -: Adapteranschlussbereich an 2
- 22 -: Rücken von 10
- 23 -: Arm von 10
- 24 -: Arm von 10
- 25 -: Kontaktfläche von 23
- 26 -: Kontaktfläche von 24
- 27 -: Kerbe in 22
- 28 -: Messkanal in 24
- 29 -: Messkanal in 23
- 30 -: Öffnung von 28
- 31 -: Öffnung von 28
- 32 -: Anschlussstutzen von 28
- 33 -: Anschlussstutzen von 29
- 34 -: Vaterstück in 3
- 35 -: Rastvorsprung
- 36 -: Rastvorsprung
- 37 -: Vaterstück an 8
- 38 -: Mutterstück an 9
- 39 -: Vaterstück an 9
- 40 -: Längskanal an 4
- 41 -: Mutterstück an 8

- P1 -: Pfeil

## Patentansprüche

1. Medizinische Vorrichtung (1) mit einem Endoskopkopf (2), der dazu ausgebildet ist, mit Endoskoprohren (4) lösbar verbunden zu werden, und mit einem Messadapter (10), wobei der Endoskopkopf (2) zwischen seinem proximalen Ende (6) und seinem distalen Ende (7) einen Längskanal (3) zur Aufnahme eines Endoskoprohrs (2) besitzt, wobei in dem Endoskopkopf (2) wenigstens ein Messkanal (13, 14) angeordnet ist, der wenigstens eine innere Anschlussöffnung (18) aufweist, die an den Längskanal (3) angeschlossen ist, und wenigstens eine äußere Anschlussöffnung (19) aufweist, die außenseitig des Endoskopkopfes (2) in einem lateralen Adapteranschlussbereich (20, 21) angeordnet ist, wobei die äußere Anschlussöffnung (19) mit wenigstens einem Messkanal (28, 29) in dem Messadapter (10) verbunden ist, der lösbar mit dem Endoskopkopf (2) gekoppelt ist, wobei der Messadapter (10) als U-förmige Klammer konfiguriert ist, mit einem Rücken (22) und mit zwei Armen (23, 24), die mit dem Rücken (22) verbunden sind, **dadurch gekennzeichnet, dass** der Messadapter Abstand vom Rücken (22) Rastvorsprünge (35, 36) aufweist,
die sich an freien Enden der federnd ausgebildeten Arme (23, 24) befinden und die nach innen zum Endoskopkopf weisen; und der Endoskopkopf eine Rastkante aufweist,
wobei die Arme (23, 24) so ausgebildet sind, dass sie beim Aufstecken des Messadapters (10) auf der Endoskopkopf (2) auseinander gebogen werden, wobei die Rastvorsprünge (35, 36) über den Adapteranschlussbereich (20, 21) des Endoskopkopfes (2) gleiten und hinter die Rastkante des Endoskopkopfes (2) schnappen, so dass die Arme (23, 24) zurückfedern und eine hinreichend dichte Verbindung zwischen dem wenigstens einen Messkanal (13, 14) im Endoskopkopf (2) und einem Messkanal (15, 16) in dem wenigstens einem Arm (23, 24) des Messadapters (10) bilden.

2. Vorrichtung nach Anspruch 1, **dadurch gekennzeichnet, dass** für die Federwirkung der Arme (23, 24) im Rücken (22) außenseitig eine Kerbe (27) angeordnet ist.

3. Vorrichtung nach Anspruch 1 oder 2, **dadurch gekennzeichnet, dass** die dichte Verbindung über eine Spaltdichtung erfolgt, indem Kontaktflächen (25, 26) der Arme (23, 24) klemmend auf den Adapteranschlussbereichen (20, 21) aufliegen.

4. Vorrichtung nach einem der Ansprüche 1 bis 3, **dadurch gekennzeichnet, dass** die Rastvorsprünge (35, 36) wulstartige Verdickungen sind.

5. Vorrichtung nach einem der Ansprüche 1 bis 4, **dadurch gekennzeichnet, dass** der Rücken (22) gebogen ist und an eine Außenkontur des Endoskopkopfes (2) angepasst ist, um als Anschlag eine Aufstecktiefe des Messadapters (10) zu begrenzen.

6. Vorrichtung nach einem der Ansprüche 1 bis 5, **dadurch gekennzeichnet, dass** der wenigstens eine Messkanal (28, 29) im Messadapter (10) im Bereich des Rückens (22) mündet.

7. Vorrichtung nach einem der Ansprüche 1 bis 6, **dadurch gekennzeichnet, dass** der Endoskopkopf (2) mit dem Endoskoprohr (4) verdrehsicher koppelbar ist, wobei die Verdrehsicherung kraftschlüssig und/oder formschlüssig ausgebildet ist, indem mindestens ein Vaterstück (34) am Endoskoprohr (4) und/oder am Endoskopkopf (2) mit wenigstens einem Mutterstück (41) am Endoskoprohr (4) und/oder am Endoskopkopf (2) in Eingriff bringbar ist.

8. Vorrichtung nach Anspruch 7, **dadurch gekennzeichnet, dass** sie eine Adapterhülse (8, 9) aufweist, wobei das endoskoprohrseitige Vaterstück (39) oder Mutterstück (41, 38) an der Adapterhülse (8, 9) ausgebildet ist, die mit einem proximalen Ende (5) des Endoskoprohrs (4) koppelbar ist.

9. Vorrichtung nach Anspruch 8, **gekennzeichnet durch** Adapterhülsen (8, 9) mit aufeinander abgestimmten Durchmessern, so dass eine im Durchmesser kleinere Adapterhülse (9) in eine im Durchmesser größere Adapterhülse (10) steckbar ist, so dass ein Endoskoprohr (4) mit kleinerem Durchmesser, das mit der kleineren Adapterhülse (9) gekoppelt ist, mit dem Endoskopkopf (2) koppelbar ist.

10. Vorrichtung nach Anspruch 8 oder 9, **dadurch gekennzeichnet, dass** die Adapterhülsen (8, 9) Messkanäle (15, 16) aufweisen, die an Messkanäle (17) in den Endoskoprohren (4) anschließbar sind und beim Eingriff in den Längskanal (3) des Endoskopkopfes (2) mit dem dortigen Messkanal (13, 14) verbunden sind.

11. Vorrichtung nach einem der Ansprüche 1 bis 10, **dadurch gekennzeichnet, dass** die Messkanäle (13, 14) im Endoskopkopf (2) diametral angeordnet sind.

## Claims

1. A medical device (1) with an endoscope head (2), which is configured to be detachably connected to endoscope tubes (4), and with a measuring adapter (10), wherein the endoscope head (2) has a longitudinal channel (3) for receiving an endoscope tube (2) between its proximal end (6) and its distal end (7), wherein at least one measuring channel (13, 14) is arranged in the endoscope head (2) which measuring channel has at least one inner connection opening (18) which is connected to the longitudinal channel (3), and at least one outer connection opening (19) which is arranged on the outside of the endoscope head (2) in a lateral adapter connection region (20, 21), wherein the outer connection opening (19) is connected to at least one measuring channel (28, 29) in the measuring adapter (10), which is detachably connected to the endoscope head (2), wherein the measuring adapter (10) is configured as a U-shaped clamp, with a back (22) and with two arms (23, 24), which are connected to the back (22), **characterised in that** the measuring adapter has latching projections (35, 36) at a distance from the back (22), which are located at free ends of the resiliently formed arms (23, 24) and which point inwardly towards the endoscope head; and
the endoscope head has a latching edge,
wherein the arms (23, 24) are configured such that they are bent apart when the measuring adapter (10) is attached to the endoscope head (2), wherein the latching projections (35, 36) slide over the adapter connection region (20, 21) of the endoscope head (2) and snap behind the latching edge of the endoscope head (2), so that the arms (23, 24) spring back and form a sufficiently tight connection between the at least one measuring channel (13, 14) in the endoscope head (2) and a measuring channel (15, 16) in the at least one arm (23, 24) of the measuring adapter (10).

2. The device according to claim 1, **characterised in that** a notch (27) is arranged on the outside of the back (22) for the spring action of the arms (23, 24).

3. The device according to claim 1 or 2, **characterised in that** the tight connection is achieved by a gap seal **in that** contact surfaces (25, 26) of the arms (23, 24) bear in a clamping manner on the adapter connection regions (20, 21).

4. The device according to any one of claims 1 to 3, **characterised in that** the latching projections (35, 36) are bead-like thickened areas.

5. The device according to any one of claims 1 to 4, **characterised in that** the back (22) is curved and is adapted to an outer contour of the endoscope head (2) in order to limit an insertion depth of the measuring adapter (10) as a stop.

6. The device according to any one of claims 1 to 5, **characterised in that** the at least one measuring channel (28, 29) opens in the measuring adapter (10) in the region of the back (22).

7. The device according to any one of claims 1 to 6, **characterised in that** the endoscope head (2) can be coupled to the endoscope tube (4) in a non-rotational manner, wherein the anti-rotation lock is configured to be non-positively and/or positively locking, **in that** at least one male piece (34) on the endoscope tube (4) and/or on the endoscope head (2) can be brought into engagement with at least one female piece (41) on the endoscope tube (4) and/or on the endoscope head (2).

8. The device according to claim 7, **characterised in that** it has an adapter sleeve (8, 9), wherein the male piece (39) or female piece (41, 38) on the endoscope tube side is formed on the adapter sleeve (8, 9), which can be coupled to a proximal end (5) of the endoscope tube (4).

9. The device according to claim 8, **characterised by** adapter sleeves (8, 9) with matching diameters, so that an adapter sleeve (9) with a smaller diameter can be plugged into an adapter sleeve (10) with a larger diameter, so that an endoscope tube (4) with a smaller diameter, which is coupled to the smaller adapter sleeve (9), can be coupled to the endoscope head (2).

10. The device according to claim 8 or 9, **characterised in that** the adapter sleeves (8, 9) have measuring channels (15, 16) which can be connected to measuring channels (17) in the endoscope tubes (4) and are connected to the measuring channel (13, 14) there when they engage in the longitudinal channel (3) of the endoscope head (2).

11. The device according to any one of claims 1 to 10, **characterised in that** the measuring channels (13, 14) are arranged diametrically in the endoscope head (2).

## Revendications

1. Dispositif médical (1) avec une tête d'endoscope (2) qui est conçue pour être connectée de manière amovible à des tubes d'endoscope (4) et avec un adaptateur de mesure (10), dans lequel la tête d'endoscope (2) comporte entre son extrémité proximale (6) et son extrémité distale (7) un canal longitudinal (3) pour recevoir un tube d'endoscope (2), dans lequel au moins un canal de mesure (13, 14) est disposé dans la tête d'endoscope (2), canal qui présente au moins une ouverture de raccordement intérieure (18) qui est raccordée au canal longitudinal (3), et au moins une ouverture de raccordement extérieure (19) qui est disposée du côté extérieur de la tête d'endoscope (2) dans une zone de raccordement d'adaptateur latérale (20, 21), dans lequel l'ouverture de raccordement extérieure (19) est connectée à au moins un canal de mesure (28, 29) dans l'adaptateur de mesure (10) qui est couplé de manière amovible à la tête d'endoscope (2), dans lequel l'adaptateur de mesure (10) est configuré en tant que pince en forme de U, avec un dos (22) et avec deux bras (23, 24) qui sont connectés au dos (22), **caractérisé en ce que** l'adaptateur de mesure présente, à distance du dos (22), des saillies d'encliquetage (35, 36) qui se trouvent aux extrémités libres des bras (23, 24) conçus de manière élastique et qui sont orientées vers l'intérieur vers la tête d'endoscope ; et
la tête d'endoscope présente un bord d'encliquetage,
dans lequel les bras (23, 24) sont conçus de sorte qu'ils sont écartés l'un de l'autre par courbure lors de la fixation de l'adaptateur de mesure (10) sur la tête d'endoscope (2), dans lequel les saillies d'encliquetage (35, 36) glissent sur la zone de raccordement d'adaptateur (20, 21) de la tête d'endoscope (2) et s'encliquettent derrière le bord d'encliquetage de la tête d'endoscope (2), de sorte que les bras (23, 24) reviennent élastiquement en arrière et forment une connexion suffisamment étanche entre le au moins un canal de mesure (13, 14) dans la tête d'endoscope (2) et un canal de mesure (15, 16) dans le au moins un bras (23, 24) de l'adaptateur de mesure (10).

2. Dispositif selon la revendication 1, **caractérisé en ce qu'**une encoche (27) est disposée dans le dos (22) du côté extérieur pour l'effet de ressort des bras (23, 24).

3. Dispositif selon la revendication 1 ou 2, **caractérisé en ce que** la connexion étanche s'effectue par l'intermédiaire d'un joint à fente, **en ce que** des surfaces de contact (25, 26) des bras (23, 24) reposent par serrage sur les zones de raccordement d'adaptateur (20, 21).

4. Dispositif selon l'une quelconque des revendications 1 à 3, **caractérisé en ce que** les saillies d'encliquetage (35, 36) sont des épaississements en forme de bourrelet.

5. Dispositif selon l'une quelconque des revendications 1 à 4, **caractérisé en ce que** le dos (22) est courbé et adapté à un contour extérieur de la tête d'endoscope (2) pour limiter, en tant que butée, une profondeur de fixation de l'adaptateur de mesure (10).

6. Dispositif selon l'une quelconque des revendications 1 à 5, **caractérisé en ce que** le au moins un canal de mesure (28, 29) débouche dans l'adaptateur de mesure (10) au niveau du dos (22).

7. Dispositif selon l'une quelconque des revendications 1 à 6, **caractérisé en ce que** la tête d'endoscope (2) peut être couplée au tube d'endoscope (4) de manière solidaire en rotation, dans lequel la sécurité antirotation est réalisée à force et/ou par complémentarité de formes, **en ce qu'**au moins une pièce mâle (34) sur le tube d'endoscope (4) et/ou sur la tête d'endoscope (2) peut être mise en prise avec au moins une pièce femelle (41) sur le tube d'endoscope (4) et/ou sur la tête d'endoscope (2).

8. Dispositif selon la revendication 7, **caractérisé en ce qu'**il présente une douille adaptatrice (8, 9), dans lequel la pièce mâle (39) ou la pièce femelle (41, 38) côté tube d'endoscope est formée sur la douille adaptatrice (8, 9) qui peut être couplée à une extrémité proximale (5) du tube d'endoscope (4).

9. Dispositif selon la revendication 8, **caractérisé par** des douilles adaptatrices (8, 9) avec des diamètres adaptés l'un à l'autre, de sorte qu'une douille adaptatrice (9) de plus petit diamètre peut être insérée dans une douille adaptatrice (10) de plus grand diamètre, de sorte qu'un tube d'endoscope (4) de plus petit diamètre, qui est couplé à la douille adaptatrice (9) plus petite, peut être couplé à la tête d'endoscope (2).

10. Dispositif selon la revendication 8 ou 9, **caractérisé en ce que** les douilles adaptatrices (8, 9) présentent des canaux de mesure (15, 16) qui peuvent être raccordés à des canaux de mesure (17) dans les tubes d'endoscope (4) et qui, lors de l'engagement dans le canal longitudinal (3) de la tête d'endoscope (2), sont connectés au canal de mesure (13, 14) qui s'y trouve.

11. Dispositif selon l'une quelconque des revendications 1 à 10, **caractérisé en ce que** les canaux de mesure (13, 14) sont disposés de manière diamétrale dans la tête d'endoscope (2).
